**Europäisches Patentamt**

**European Patent Office**

**Office européen des brevets**

(19)

(11) Veröffentlichungsnummer : **0 267 544 B1**

(12) # EUROPÄISCHE PATENTSCHRIFT

(45) Veröffentlichungstag der Patentschrift :
05.06.91 Patentblatt 91/23

(51) Int. Cl.⁵ : **G01L 1/20**, G01L 9/00

(21) Anmeldenummer : 87116357.2

(22) Anmeldetag : 06.11.87

(54) **Druckmesselement.**

(30) Priorität : 12.11.86 DE 3638641

(43) Veröffentlichungstag der Anmeldung :
18.05.88 Patentblatt 88/20

(45) Bekanntmachung des Hinweises auf die
Patenterteilung :
05.06.91 Patentblatt 91/23

(84) Benannte Vertragsstaaten :
AT BE CH DE ES FR GB IT LI LU NL SE

(56) Entgegenhaltungen :
EP-A- 0 145 532
EP-A- 0 161 895
EP-A- 0 218 465
US-A- 4 555 954

(56) Entgegenhaltungen :
PATENT ABSTRACTS OF JAPAN, Band 9, Nr.
195, (P-379)(1918) 13. August 1985 & JP, A,
60060516
PATENT ABSTRACTS OF JAPAN, Band 11, Nr.
22 (P 538)(2469), 21. Januar 1987 & JP A
61195324

(73) Patentinhaber : Richter, Dietrich H.
Birkenfeld Nr. 83
W-8530 Neustadt a.d. Aisch (DE)

(72) Erfinder : Mollenhauer, Christian, Prof. Dr.-Ing.
Beroldinger Strasse 14
W-7732 Niedereschach (DE)

(74) Vertreter : Brose, Manfred, Dr.
Pellergasse 45
W-8500 Nürnberg 50 (DE)

## Beschreibung

Die Erfindung betrifft ein Druckmeßelement zur elektrischen Messung eines kleinen mechanischen flächenförmigen Druckes, insbesondere zur Ermittlung des Auflagedruckes einer liegenden oder stehenden Person, bestehend aus ein oder zwei elektrisch leitfähigen Silicon-Kautschuk (LSK) Elementen zwischen zwei metallischen Kontakten.

Aus der europäischen Patentanmeldungsschrift EP-A-0 161 895 ist ein Druckmeßelement bekannt, daß aus zwei sich kreuzenden Leitern besteht, wobei jeder Leiter von einem faserigen Widerstandsmaterial umgeben ist. Dieses Widerstandsmaterial kann auch LSK sein. Die einzelnen Druckmeßelemente bestehen jedoch nicht aus homogenen zylindrischen LSK-Elementen, sondern haben eine faserige, textile Struktur. Homogene LSK-Elemente sind aber für genauere Messungen von großer Bedeutung.

Der Erfindung liegt daher die Aufgabe zugrunde, ein aus LSK-Elementen bestehendes Druckmeßelement zu schaffen, dessen Empfindlichkeit für Druckmessungen möglichst groß und dessen Aufbau möglichst einfach ist.

Diese Aufgabe wird nach der Erfindung gelöst durch ein streifenförmiges LSK-Element mit einer Kontaktfolie auf einer Seite und ein an der anderen Seite anliegendes, um ein Federrohr herumgelegtes zweites LSK-Element, wobei die Kontaktierung entweder über das Federrohr oder über eine dazwischenliegende Kontaktfolie erfolgt.

Eine bevorzugte Anwendungsform des vorstehend beschriebenen Druckmeßelementes erfolgt in einer Meßmatte, die aus einer Vielzahl, eine Meßmatrix bildender Druckmeßelemente besteht. Vorzugsweise ist in einer Meßmatrix vor jedes einzelne Druckmeßelement der Matrix eine Diode oder ein Transistor geschaltet.

In den Zeichnungen zeigen :

Fig. 1 einen Schnitt durch ein aus zwei sich kreuzenden streifenförmigen LSK Elementen bestehendes Druckmeßelement,

Fig. 2 (nicht beanspsacht) einen Schnitt durch ein aus zwei sich kreuzenden zylinderförmigen LSK Elementen bestehendes Druckmeßelement,

Fig. 3 einen Teil einer Meßmatrix ohne Sperrdioden und

Fig. 4 einen Teil einer Meßmatrix mit Sperrdioden.

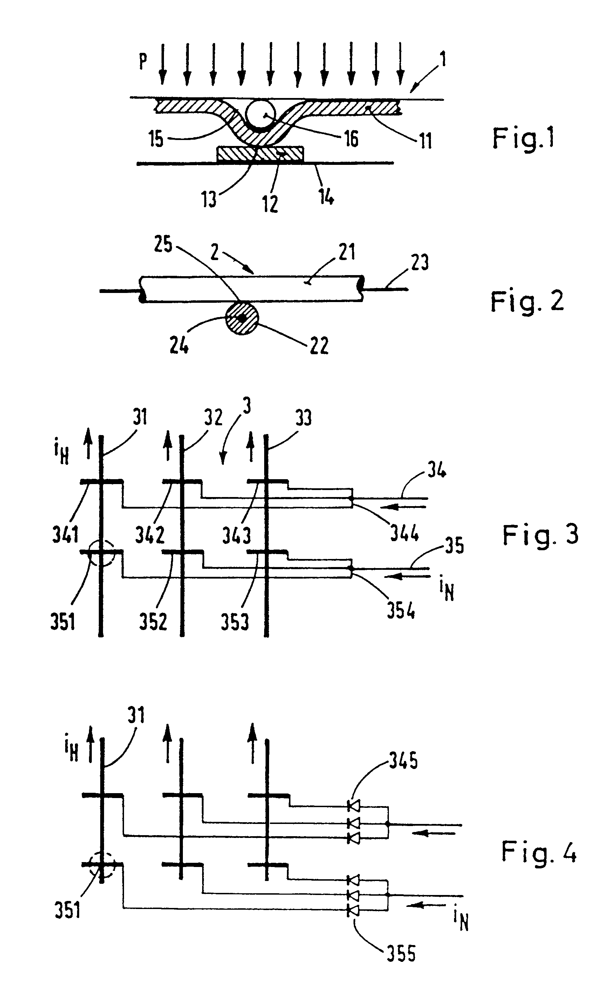

In Figur 1 ist ein Schnitt durch ein Druckmeßelement 1 wiedergegeben, das aus zwei sich kreuzenden streifenförmigen LSK Elementen 11, 12 besteht. Das Druckmeßelement 1 dient zur elektrischen Messung eines kleinen mechanischen flächenförmigen Druckes, so wie dieser durch die Pfeile P angedeutet ist. Vorzugsweise dient das Druckmeßelement 1 zur Ermittlung des Auflagerdruckes einer liegenden oder stehenden Person. Auf die Außenseite des LSK Streifens 11 ist eine Metallfolie 15 und auf die Außenseite des LSK Streifens 12 eine Metallfolie 14 aufvulkanisiert, durch die das Druckmeßelement 1 mit Auswertungsgeräten für die elektrischen Signale verbunden werden kann. Das elektrische Signal, das durch die Änderung des Übergangswiderstandes hervorgerufen wird, wird bei dem Druckmeßelement 1 durch zwei Effekte erzeugt, nämlich einmal durch eine Vergrößerung der Kontaktfläche zwischen den beiden LSK Elementen 11, 12, die sich im unbelasteten Zustand nur in einer Kontaktlinie 13 berühren und zweitens durch eine Widerstandsänderung innerhalb jedes einzelnen LSK Elementes 11, 12 infoge der Materialverdichtung. Um die elastische Verformung der beiden LSK Elemente 11, 12 zu begrenzen, ist das LSK Element 11 um ein Federrohr 16 herumgelegt. Als Federrohr 16 dient hierbei ein in Längsrichtung geschlitztes Rohr. Durch den Schlitz (nicht gezardnet) wird ein Teil des Auflagedruckes vom Rohr aufgenommen, so daß eine zu hohe Druckbelastung der LSK Elemente 11, 12 vermieden wird. Dieses kann notwendig werden, da in einem Widerstands- / Verformungsdiagramm der Widerstandswert in Abhängigkeit von der Verformung eine starke Hysterese zeigt.

In Figur 2 ist ein Druckmeßelement 2 wiedergegeben, das aus zwei sich kreuzenden zylinderförmigen LSK Elementen besteht. Jedes LSK Element besteht aus einem zylinderförmigen LSK Rohr 21, 22 mit einer zentralen metallischen Seele 23, 24. Durch die Kreuzung beider LSK Elemente haben diese nur einen Berührungspunkt 25. Die Ansprechempfindlichkeit des Druckmeßelementes 2 ist daher höher als die des Druckmeßelementes 1.

Die vorstehend beschriebenen Druckmeßelemente 1, 2 werden vorzugsweise in einer großen Anzahl in Form einer regelmäßigen Meßmatrix in einer Meßmatte zusammengefaßt. Die Meßmatte kann Körpergröße haben, um die Auflagedruckverteilung eines liegenden Menschen zu ermitteln, sie kann aber auch die Form einer Schuheinlegesohle haben, um die Druckverteilung in der Fußsohle eines stehenden Menschen zu ermitteln. Die Meßmatte besteht vorzugsweise aus einem Oberteil und einem Unterteil, wobei in die Innenfläche des Oberteiles Längsnuten und in die Innenfläche des Unterteiles Quernuten zur Aufnahme zylinderförmiger LSK Rohrelemente eingefräßt sind.

In Figur 3 ist der Ausschnitt aus einer Meßmatrix 3 wiedergegeben, der aus drei Spalten mit den LSK Elementen 31, 32, 33 und zwei Zeilen 34, 35 besteht. Jede Zeile 34, 35 besteht ihrerseits beispielsweise aus drei LSK-Elementen 341, 342, 343 oder 351, 352, 353. Es versteht sich, daß jeder Kreuzungspunkt entsprechend den in den Figuren 1 und 2 beschriebenen Druckmeßelementen 1 oder 2 aufgebaut ist. Die LSK Elemente 341, 342, 343 sind jeweils zu einem Leiter 34 zusammengefaßt. Entsprechendes gilt für die LSK Elemente 351, 352, 353, die zu einem Leiter 35 zusammengefaßt sind.

Wird nun durch eine entsprechende Schaltung der aus den Leitern 31, 35 bestehende Knotenpunkt 351 zur Durchführung einer Druckmessung an eine elektronische Auswerteinrichtung gelegt, dann fließt nicht nur zwischen den Leitern 31, 35 der Hauptstrom $i_H$, sondern über die Brücken 344, 354 auch noch der Nebenstrom $i_N$. Hierdurch erfolgt eine Verfälschung des Meßergebnisses.

In Figur 4 sind dagegen die LSK Elemente jeder Teile über Dioden oder Transistoren 345, 355 miteinander verbunden. Durch die Dioden oder Transistoren 345, 355 werden die Nebenströme $i_N$ unterbrochen. Es kann nur noch der zu dem Matrix- oder Knotenpunkt 351 gehörende Hauptstrom $i_H$ fließen, der das richtige Meßergebnis repräsentiert.

## Ansprüche

1. Druckmeßelement (1) zur elektrischen Messung eines kleinen mechanischen flächenförmigen Druckes, insbesondere zur Ermittlung des Auflagedruckes einer liegenden oder stehenden Person, bestehend aus zwei sich kreuzenden metallischen Kontakten (14, 15), zwischen denen sich ein elektrisch leitfähiges Silikon-Kautschuk (LSK) Element (11, 12) befindet, gekennzeichnet durch ein streifenförmiges LSK-Element (12) mit einer Kontaktfolie (14) auf einer Seite und ein an der anderen Seite anliegendes, um ein Federrohr (16) herumgelegtes zweites LSK-Element (11), wobei die Kontaktierung entweder über das Federrohr (16) oder über eine dazwischenliegende Folie (15) erfolgt

2. Druckmeßelement (1) nach Anspruch 1 in einer matrixförmigen Anordnung, dadurch gekennzeichnet, daß vor jedes einzelne Druckmeßelement (351) der Matrix eine Diode oder ein Transistor (345, 355) geschaltet ist.

## Claims

1. Pressure-measuring element (1) for the electrical measurement of a small mechanical areal pressure, in particular for ascertaining the bearing pressure of a lying or standing person, the element consisting of two mutually crossing metallic contacts (14, 15), between which an electrically conductive silicone rubber element (11, 12) is disposed, characterised by a strip-shaped conductive silicone rubber element (12) with a contact foil (14) at one side and a second conductive silicone rubber element (11) laid around a resilient tube (16) and lying against the other side, wherein the contact takes place either by way of the resilient tube (16) or by way of a foil (15) lying therebetween.

2. Pressure-measuring element (1) according to claim 1 in a matrix-shaped arrangement, characterised thereby, that a diode or a transistor (345, 355) is connected in front of each individual pressure-measuring element (351) of the matrix.

## Revendications

1. Elément de mesure de pression (1) pour la mesure électrique d'une faible pression mécanique superficielle, en particulier pour la détermination de la pression d'appui d'une personne couchée ou debout, composé de deux contacts métalliques qui se croisent (14, 15), entre lesquels se trouve un élément en caoutchouc de silicone conducteur (CSC) (11, 12), caractérisé par un élément en CSC en forme de bande (12) muni sur une face d'une feuille de contact (14) et un second élément en CSC (11) disposé autour d'un ressort tubulaire (16), en contact avec l'autre face, la prise de contact s'effectuant soit par le tube élastique (16), soit par une feuille interposée (15).

2. Elément (1) selon la revendication 1, dans une disposition matricielle, caractérisé en ce qu'avant chaque élément de mesure de pression individuel (351) de la matrice est branché une diode ou un transistor (345, 355).

P

Fig.1

Fig. 2

Fig. 3

Fig. 4